(19)

Europäisches Patentamt

European Patent Office

Office européen des brevets

(11)  **EP 4 344 845 A1**

(12)  **EUROPEAN PATENT APPLICATION**

(43) Date of publication:
**03.04.2024 Bulletin 2024/14**

(21) Application number: **23200679.1**

(22) Date of filing: **28.09.2023**

(51) International Patent Classification (IPC):
**B29C 33/42** *(2006.01)*      **B29C 39/26** *(2006.01)*
**B29C 39/42** *(2006.01)*      **C25D 1/00** *(2006.01)*
**G01N 33/554** *(2006.01)*    **G01N 33/92** *(2006.01)*

(52) Cooperative Patent Classification (CPC):
**B29C 33/424; B29C 39/26; B29C 39/42;**
**C25D 1/003; G01N 33/554; G01N 33/92**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **30.09.2022  KR 20220125837**
**14.08.2023  KR 20230106268**

(71) Applicant: **Korea Institute of Science and Technology**
**Seoul 02792 (KR)**

(72) Inventors:
• **KIM, Tae Song**
  02792 Seoul (KR)
• **KANG, Dong Hyun**
  02792 Seoul (KR)
• **KIM, Bong Kyu**
  02792 Seoul (KR)

(74) Representative: **Handley, Matthew Edward et al**
**Venner Shipley LLP**
**200 Aldersgate**
**London EC1A 4HD (GB)**

(54)  **METHOD FOR PREPARING SOLVENT-FREE 3D BIOLOGICAL BILAYER MEMBRANE STRUCTURE IN PHYSIOLOGICAL SOLUTION AND 3D BIOLOGICAL BILAYER MEMBRANE STRUCTURE USING THE SAME**

(57)    The present invention relates to a method for preparing a 3D biological bilayer membrane structure in a physiological buffer solution and a 3D biological bilayer membrane structure using the same, and more particularly, to a method for preparing a 3D biological bilayer membrane structure that is tightly sealed even under physiological ionic conditions by applying pressure during electroformation to improve a membrane fusion function, and a 3D biological bilayer membrane structure using the same.

【FIG. 1】

**EP 4 344 845 A1**

**Description**

CROSS-REFERENCE TO RELATED APPLICATION

This application claims priority to and the benefit of Korean Patent Application No. 2022-0125837, filed on September 30, 2022, and Korean Patent Application No. 2023-0106268, filed on August 14, 2023, the disclosure of which is incorporated herein by reference in its entirety.

BACKGROUND

**1. Field of the Invention**

The present invention relates to a method for preparing a solvent-free 3D biological bilayer membrane structure in a physiological solution and a 3D biological bilayer membrane structure using the same, and more particularly, to a method for preparing a 3D biological bilayer membrane structure that is tightly sealed even under physiological ionic conditions by applying pressure during electroformation to improve a membrane fusion function, and a 3D biological bilayer membrane structure using the same.

**2. Discussion of Related Art**

Cells perform a function for maintaining life phenomena such as sensing and regulating external changes and communication between intracellular and extracellular information. At the core of this cellular functionality lies an amphiphilic bilayer having a thickness of about 4 to 10 nm, which incorporates membrane proteins, including ion channel proteins.

Artificial cell technology, focused on reconstituting cell membranes, membrane proteins, and intracellular compartments, has broad applications spanning multiple domains such as 1) the investigation on life phenomena, 2) the exploration of brain function signaling, 3) novel drug screening methodologies, and 4) new dimension of molecular level sensors.

Compartmentalization using artificial cell membranes is essential to mimic cellular systems. As standard requirements for model cell membranes, artificial cell membranes need to provide the same sealing capabilities as original cell membranes, and need to be generated and operates under physiological conditions similar to those *in vivo* to ensure sufficient stability that enables various biological/engineering attempts using biological processes similar to those of cells.

Regarding artificial cell membrane technology, giant unilamellar vesicles (GUVs) produced by electroformation are the most suitable option because they are solvent-free and relatively stable compared to two-dimensional bilayer lipid membranes (BLMs) and can embody a surface area and volume similar to those of cells.

Recently, attempts have been made to prepare artificial cell membrane structures in various 3D forms immobilized on a non-flat substrate using electroformation. Takeuchi *et al.* of the University of Tokyo manufactured a dome-shaped and vesicle-shaped lipid structure array using 1 μm-deep microwells on an ITO glass surface. However, the dome-shaped structure has imperfect sealing and thus cannot completely isolate the inside and outside of the lipid structure, and the vesicle-shaped structure has a disadvantage of detachment from the substrate. Further, the Wostein group of Pennsylvania State University prepared giant unilamellar vesicles (GUVs) with a size of 10 to 70 μm by applying an electric field for electroforming after transferring lipid stacks as an array on an ITO glass substrate using a hydrogel stamp. However, since the lipid bilayer structures immobilized on the substrate cannot be maintained, the floating GUVs are limited only to their role as an existing container.

An electric field applied during electroformation induces oscillation of the lipid stack due to the electroosmotic behavior of the medium and thus is very effective for causing initial membrane expansion and fusion. However, since the occurrence of membrane expansion and swells is random and uncontrollable, GUVs are generated with a polydisperse size distribution. Also, the generation of GUVs under ionic solutions is difficult because electroformation is weakened by ions that interfere AC electric field and reduce membrane expansion and fusion, especially under physiological ionic conditions. In general, even in the case of non-ionic media, membranes do not fuse easily under normal circumstances due to the presence of repulsive force between the hydrophilic polar heads of each lipid membrane.

Since 3D lipid structures prepared through conventional attempts have not overcome 1) electroformation using non-ionic media, 2) inability to maintain a fixed 3D form, and 3) inability to completely isolate (sealing) the inside and outside, which are limitations in the previous artificial cell membrane preparation technology, only basic level research is being carried out, and they are a long way from industrial use.

Therefore, in order to be created and function in an environment similar to cells, there is a need for developing a technique capable of preparing a 3D artificial cell membrane structure prepared and maintained in a physiological solution in various sizes and shapes, partitioning the inside/outside by perfectly sealing a prepared 3D structure, and maintaining the 3D structure firmly and stably for a longer period of time.

SUMMARY OF THE INVENTION

Thus, the present invention has been devised to meet the aforementioned needs, and an object of the present invention is to provide a method for preparing a 3D biological bilayer membrane structure, which is prepared in a physiological buffer solution and tightly sealed, which can prepare a highly stable 3D structure with a desired size and shape by regulating the amplitude and frequency of an electric field during the electroforming process and applying a controlled pressure, and a 3D biological bilayer membrane structure prepared by the above method.

The present invention provides a method for preparing a three-dimensional (3D) biological bilayer membrane structure, the method including: preparing a microwell array in which multiple microwells are formed on a substrate (Step a), coating the inside of the microwells with an artificial biological membrane material (Step b), and injecting a buffer solution onto the microwell coated with the biological membrane material and applying an electric field and pressure (Step c).

The microwell may have a diameter in a range of 1 to 20 $\mu$m.

The microwell may have an aspect ratio (aspect ratio = depth/diameter) in a range of 0.2 to 10.0.

The artificial biological membrane material may be coated by a spin-casting method.

The artificial biological membrane material may include one or more of a lipid or a block copolymer.

The buffer solution may be a physiological solution.

The physiological solution may include one or more selected from the group consisting of a solution containing $H^+$, $K^+$, $Na^+$ or $Cl^-$ ions, 4-(2-hydroxyethyl)-1-piperazineethanesulfonic acid (HEPES), tris(hydroxymethyl)aminomethane (Tris) or Dulbecco's phosphate-buffered saline (DPBS).

The electric field may be applied by applying an electric field using an electroformation method.

The pressure may be applied by external equipment, or by a method of generating relative pressure in the microwells by limiting the movement of an aqueous solution on a microwell array substrate inserted in a microchannel (a method of generating pressure on a microwell by interfering with the flow of fluid on a microwell array substrate).

The method of generating relative pressure inside the microwell by limiting the movement of an aqueous solution may be performed by a method of generating pressure on the microwell by disposing a hydrogel block.

The hydrogel may include polyethylene glycol dimethacrylate (PEGDMA).

The hydrogel may be a mixture of PEGDMA 1000 and PEGDMA 3400.

Step c may be performed under conditions of a frequency of 5 Hz to 10 MHz and a pressure of 300 kPa or less. More specifically, Step c may be performed under a pressure condition of 150 kPa or less and 300 kPa or less when the biological membrane material is a lipid and a block copolymer, respectively.

In addition, the present invention provides a 3D biological bilayer membrane structure prepared by the method.

The structure may maintain 90% or more stability for at least 17 days in the case of a lipid, and may maintain 90% or more stability for at least 38 days when pressure is applied through a hydrogel block.

The structure may maintain 90% or more stability for at least 50 days in the case of a block copolymer.

Furthermore, the present invention may provide a biomimetic biosensor or bioreactor including a 3D biological bilayer membrane structure prepared by the method.

BRIEF DESCRIPTION OF THE DRAWINGS

The above and other objects, features and advantages of the present invention will become more apparent to those of ordinary skill in the art by describing in detail exemplary embodiments thereof with reference to the accompanying drawings, in which:

FIG. 1 shows an example illustratively describing a 3D biological bilayer membrane structure (3DFLB: 3D freestanding lipid bilayer) that can improve the controllability of membrane fusion under physiological ionic conditions by simultaneously applying pressure and an electric field prepared according to the present invention to prepare the 3D biological bilayer membrane structure;

FIG. 2 shows an example of the process of precisely controlling and evaluating the hydraulic pressure applied to a microchannel by a syringe pump;

FIG. 3 shows an example of a microwell array substrate;

FIG. 4 is a schematic view of the step of patterning lipids into microwells;

FIG. 5 shows an example of promoting biological membrane fusion in the electroformation step according to the applied pressure;

FIG. 6 shows an example of the process of preparing a 3D biological bilayer membrane structure (3DFLB: 3D freestanding lipid bilayer) that can improve the controllability of membrane fusion under physiological ionic conditions by simultaneously applying pressure and an electric field prepared according to the present invention and an example of the size distribution of the prepared 3D biological bilayer membrane structure;

FIG. 7 is a schematic view of the tight sealing of a 3D biological bilayer membrane structure and an example of the correlation between the 3D biological bilayer membrane structure according to a rehydration solution and AC frequency and applied pressure;

FIG. 8 shows an example of the regulation of membrane fusion of a biological bilayer membrane structure according to changes in pressure in the electroformation step in a physiological solution;

FIG. 9 shows an example of a tightly sealed three-dimensional biological bilayer membrane structure in a physiological solution;

FIG. 10 shows an example of endocytosis or molecular transport mimicked through nanopore formation by melittin injection and membrane fusion by pressure based on a 3D biological bilayer membrane structure as an application platform; and

FIG. 11 is a schematic view of in-chip pressure control through a hydrogel block located over a microwell template and an example of pressure control through the regulation of the composition of the hydrogel block.

DETAILED DESCRIPTION OF EXEMPLARY EMBODIMENTS

Since the present invention may be modified into various forms and include various exemplary embodiments, specific exemplary embodiments will be illustrated in the drawings and described in detail in the Detailed Description. However, the description is not intended to limit the present invention to specific exemplary embodiments, and it is to be understood that all the changes, equivalents, and substitutions belonging to the spirit and technical scope of the present invention are included in the present invention. When it is determined that the detailed description of the related publicly known art in describing the present invention may obscure the gist of the present invention, the detailed description thereof will be omitted.

The terms used in the present application are used only to describe specific embodiments, and are not intended to limit the present invention. Singular expressions include plural expressions unless the context clearly indicates otherwise. In the present application, the term "include" or "have" is intended to indicate the presence of a characteristic, number, step, operation, constituent element, part or any combination thereof described in the specification, and it should be understood that the possibility of the presence or addition of one or more other characteristics or numbers, steps, operations, constituent elements, parts or any combination thereof is not precluded.

Terms such as first and second may be used to describe various constituent elements, but the constituent elements should not be limited by these terms. The above terms are used only to distinguish one constituent element from another constituent element.

The present invention provides a method for preparing a 3D biological bilayer membrane structure by controlling membrane fusion through the interplay of pressure and an electric field.

By controlling pressure while applying an electric field using an electroformation method, membrane fusion may be enhanced, enabling the preparation of a 3D biological bilayer membrane structure by improving the fusion of the biological membrane even under physiological ionic conditions. Further, precise regulation of pressure and electric field allows to preparation of a tightly sealed three-dimensional biological bilayer membrane structure with a controlled size and shape.

When membrane fusion is controlled through the interaction between pressure and an electric field to prepare a 3D biological bilayer membrane structure, the stability of the lipid three-dimensional biological bilayer membrane structure may be extended to approximately 17 days or more. Particularly, stability may be extended to approximately 38 days or more, when pressure is controlled using a hydrogel block (in-chip pressure control). Accordingly, various biological applications of a fragile lipid membrane may become feasible.

In one exemplary embodiment of the present invention, a 3D biological bilayer membrane structure, immobilized on a substrate under physiological ionic conditions, was prepared by simultaneously applying hydraulic pressure and an electric field in a microfluidic channel. Through this example, based on membrane fusion that occurred irregularly between membrane expansion and an expanded membrane controlled through electroformation in microwell arrays within the microfluidic channel, the present inventors confirmed an effect in which hydraulic pressure promotes membrane fusion in the microwell array, and a method of controlling the shape and size of a 3D biological bilayer membrane structure and improving sealing characteristics by combining hydraulic pressure and an electric field. In addition, the present inventors confirmed the possibility of regulating the pressure in microfluidic channels using a hydrogel block with a controlled pore size instead of regulating pressure through an external device. Furthermore, the present inventors demonstrated that microfluidic channels can deliver reagents into the artificial cell membrane through regulated pressure by allowing hydraulic pressure to promote membrane fusion between the 3D biological bilayer membrane structure immobilized on the substrate and injected small unilamellar vesicles (SUVs) to mimic in vivo endocytosis.

Specifically, the method for preparing a 3D biological bilayer membrane structure includes: preparing a microwell array in which multiple microwells are formed on a substrate (Step a), coating the inside of the microwells with an artificial biological membrane material (Step b), and injecting a buffer solution onto the microwell coated with the biological membrane material, and then applying an electric field and pressure (Step c).

Preparing microwell array (Step a)

This is a step of preparing a microwell array in which multiple microwells are formed on a substrate.
The microwell array may consist of multiple microwells formed on a substrate, and a 3D structure is prepared using each microwell.
The substrate includes, without particular limitation, all those known in the art as a support for forming a 3D structure thereon.
For example, the substrate may be a plate or substrate including silicon.
For example, a silicon substrate is prepared and a photosensitive polymer (photoresist) is coated thereon to form a photosensitive polymer film. Then, a chrome mask is placed on the photosensitive polymer film and exposure is performed through a lithography process. Subsequently, the photosensitive polymer film may be patterned through development to form a microwell array. The photosensitive polymer is not particularly limited as long as it can form a microwell array with a suitable thickness.
The substrate is a support for forming a 3D biological membrane structure thereon, and the microwell is a space for forming a biological membrane layer by filling a solution including a biological membrane material therein. The shape of the microwells is not particularly limited. For example, the microwell may have a square or cylindrical shape, and may also have a square cross-section, but is not limited thereto. The microwells may be arranged at regular intervals on the substrate to form a microwell array.
The microwells may have a diameter in a range of 1 to 20 $\mu$m, more preferably in a range of 8 to 12 $\mu$m.
The spacing (pitch) between the plurality of microwells may be regulated to be in a range of 10 to 100 $\mu$m, but is limited thereto. The pitch is sufficient as long as it is greater than or equal to a pitch at which the structures formed in each microwell do not interfere with each other.
The microwells may have an aspect ratio (aspect ratio = depth/diameter) in a range of 0.2 to 10.0, more preferably in a range of 0.5 to 10.0.
In order to prepare a 3D biological membrane structure, a structure that can promote membrane fusion during the rehydration process of a dried biological membrane material is required, and it is good to have the aspect ratio as described above.

Coating inside of microwells with artificial biological membrane material (Step b)

In this step, a biological membrane material solution is injected into the microwells and then dried to form an artificial biological membrane layer.
The biological membrane material may include a lipid, a mixture of a lipid and a cell membrane composition other than the lipid, a block copolymer, a mixture of a block copolymer and a lipid, and the like.
The solution may include a biological membrane material such as a lipid or block copolymer at a concentration in a range of 1 to 50 mM, preferably in a range of 5 to 30 mM. The solvent is not particularly limited as long as it is an organic solvent such as trichloroethylene, chloroform, or methanol, which is capable of dissolving a biological membrane material such as a lipid or block copolymer.
The solution is not particularly limited as long as it is a solution capable of including liposomes or polymersomes or small vesicles (SVs), such as deionized water (DI water) including liposomes or polymersomes or small vesicles (SVs) consisting of small lipids and polymers, or mixtures thereof.
The biological membrane material solution may further include a biological membrane material labeled with fluorescence for later confirmation with a fluorescence microscope. As a fluorescent biological membrane material, it is possible to use a material in which a fluorescent material, which emits green fluorescence (NBD) and/or red fluorescence (rhodamine B), is attached to the head portion of a lipid called 1,2-dioleoyl-sn-glycero-3-phosphoethanolamine (DOPE), a block copolymer terminated with a fluorescent material, and the like. For example, 1,2-dioleoyl-sn-glycero-3-phosphoethanolamine-N  -(7-nitro-2-1,3-benzoxadiazol-4-yl (NBD-PE)  and/or  1,2-dioleoyl-sn-glycero-3-phosphoethanolamine-N-(lissamine rhodamine B sulfonyl) (Liss Rhod PE) may be used in an amount ranging from 0.1 to 10 mol%, preferably ranging from 0.3 to 1.0 mol% with respect to the biological membrane material solution. For example, rhodamine B terminated poly(butadiene-b-ethylene oxide) may be used in an amount ranging from 0.1 to 10 mol%, preferably ranging from 0.3 to 1.0 mol%, with respect to the biological membrane material solution.
After the biological membrane material solution is injected into the microwell, a biological membrane layer may be formed through a drying step.
The drying may be performed at a temperature in a range of -10°C to -80°C, and it is more preferable to perform the drying at a pressure in a range of 1 mTorr to 10 mTorr for 5 to 20 hours. For example, the biological membrane material may be dried in a freeze dryer. It is preferable to dry the biological membrane material at a temperature lower than the transition temperature (-17°C) of DOPC (lipid), and it is more preferable to dry the biological membrane layer in a range of -20°C to -70°C in order to prevent the lipid or block copolymer from being denatured. In addition, it is better to dry the biological membrane material at 5 mTorr for 6 hours or more to evaporate all the solvent molecules used to dissolve the lipid or block copolymer.

In order to regulate the desired shape and size of the 3D biological bilayer membrane structure immobilized on the substrate, it is essential to selectively inject and dry the biological material inside the microwells.

For this purpose, it is important to form an appropriate contact angle, and a desired contact angle may be formed by performing chemical treatment (such as $O_2$ plasma or silane) on the surface of the substrate.

The chemical treatment may be performed by a cleaning method (such as piranha cleaning), but is not limited thereto.

In a specific embodiment of the present invention, a solution including a biological membrane material was dropped onto a substrate with an appropriately regulated contact angle, and then the biological membrane material was selectively injected only inside the microwells by a spin-casting method.

Thereafter, the biological membrane material was dried using a freeze dryer at room temperature and -70°C to evaporate the solution, leaving only the biological membrane material. Microwell substrates including the dried biological membrane material may be stored in a freeze dryer at -70°C until rehydration to prevent the material from being denatured.

Injecting buffer solution, and then applying electric field and pressure (Step c)

This step is a step of preparing a 3D biological bilayer membrane structure by injecting a buffer solution into the biological membrane material that has been selectively coated and dried inside the microwell, and may prepare a 3D biological bilayer membrane structure that is tightly sealed even under physiological ionic conditions. Further, when pressure and an electric field are regulated, it is possible to prepare a tightly sealed three-dimensional biological bilayer membrane structure with a controlled size and shape.

The buffer solution is for hydrating the coated and dried biological membrane layer.

The buffer solution may include a physiological solution. Here, the physiological solution may include one or more selected from the group consisting of a solution containing $H^+$, $K^+$, $Na^+$ or $Cl^-$ ions, 4-(2-hydroxyethyl)-1-piperazineethanesulfonic acid (HEPES), tris(hydroxymethyl)aminomethane (Tris) or Dulbecco's phosphate-buffered saline (DPBS).

The present Step c is for preparing a 3D biological bilayer membrane structure that is tightly sealed even under physiological ionic conditions by applying pressure during electroformation to improve the membrane fusion function, and may be performed under the conditions of a frequency of 5 Hz to 10 MHz and a pressure of 300 kPa or less (or 10 Pa to 300 kPa). More specifically, Step c may be performed under a pressure condition of 150 kPa or less and 300 kPa or less, when the biological membrane material is a lipid and a block copolymer, respectively. More preferably, Step c may be performed under a pressure condition of 100 to 130 kPa and 100 to 200 when the biological membrane material is a lipid and a block copolymer, respectively, at a frequency of 500 Hz to 100 kHz.

When the pressure is out of the above range, the structure may not be properly sealed, or it may be difficult to produce a 3D biological bilayer membrane structure.

The block copolymers may be configured in various ways. For example, the block copolymer may include polybutadiene-polyethyleneoxide (PBd-PEO), but is not limited thereto.

According to the present step, the fusion of the biological material may be promoted or regulated by breaking away from the methods that have been performed for the past decades, which rely on the regulation of the electric field, and applying a precisely controlled pressure other than an alternating electric field in the rehydration step of the biological membrane material in the microwells.

The electric field may be applied by applying an electric field using an electroformation method.

The pressure may be applied by external equipment, or by a method of applying relative pressure to the inside of the microwells by limiting the movement of an aqueous solution on a microwell array substrate inserted in a microchannel (a method of generating pressure on a microwell by interfering with the flow of fluid on a microwell array substrate), for example, a method of generating pressure in the microwells by disposing a hydrogel block.

Here, the hydrogel may be polyethylene glycol dimethacrylate (PEGDMA), preferably a mixture of different hydrogels. More preferably, the hydrogel may be a mixture of PEGDMA 1000 and PEGDMA 3400. More specifically, a desired pressure may be applied by mixing PEGDMA 1000 and PEGDMA 3400 at a weight ratio of 10:5 to 5: 10 to adjust the pore size.

When a desired pressure is applied by regulating the mixing composition of the hydrogel block without an additional external device, it is possible to prepare a 3D biological bilayer membrane structure with higher stability and lifetime.

According to a specific embodiment of the present invention, it was confirmed that the 3D biological bilayer membrane structure made of lipid material prepared by applying pressure maintains about 90% of the structure for about 17 days, and in particular, when a hydrogel block was applied without an additional external device, about 90% of the structure was maintained up to about 38 days.

Since the method for preparing a 3D biological bilayer membrane structure according to the present invention can prepare an array without any solvent from a lipid or a mixture of a lipid and a cell membrane composition other than the lipid, or a block copolymer, or a mixture of a block copolymer and a lipid and can achieve tight sealing, the 3D biological bilayer membrane structure is stable and has a very long lifetime, and thus is industrially useful.

Furthermore, the 3D biological bilayer membrane structure prepared by the preparation method of the present invention is an array structure, which is tightly sealed even in a physiological solution and is in the form of being immobilized on a solid substrate, and can be applied to various fields such as new drug screening, artificial olfactory sensors, and ultrasensitive biosensors. More specifically, the 3D biological bilayer membrane structure prepared by the preparation method of the present invention can be used as a biomimetic biosensor, a biomimetic bioreactor, or the like.

Hereinafter, the present invention will be described in more detail through an example. The objects, features and advantages of the present invention will be readily understood through the following description. The present invention is not limited to the example described herein, and may be implemented in different forms. The example introduced herein is provided such that the idea of the present invention can be sufficiently conveyed to a person with ordinary skill in the art to which the present invention pertains. Therefore, the present invention should not be limited by the following example.

**Example**

<Method>

Manufacture of microwell array on silicon substrate

A microwell array was manufactured on a silicon substrate using an epoxy-based negative photoresist SU-8. An Si wafer was spin-coated with SU-8 to a thickness of 8 $\mu$m. SU-8 was exposed to UV light, then developed in a SU-8 developer and rinsed with IPA and DI water to manufacture a microwell array.

Lipid patterning in microwell array

For fluorescence observation, an appropriate amount of chloroform was added to a vial containing DOPC containing 1 mol% Rhod-PE to obtain a 17 mM lipid solution. After the chloroform was evaporated, the dried lipid membrane was hydrated with deionized water (DI water) under gentle stirring. A suspension of the hydrated lipid membrane including micrometer-sized vesicles with a multilayered structure was obtained and then extruded through a polycarbonate membrane filter. The SUV solution was stored at 4°C for future use. The prepared SUV solution was spin-casted onto the SU-8 microwell template to coat only the inside of the microwell, and dried in a freeze dryer for 6 hours or more.

Formation of 3DFLB, which is 3D biological bilayer membrane (or 3D independent lipid bilayer) by hydraulic pressure-assisted electroformation

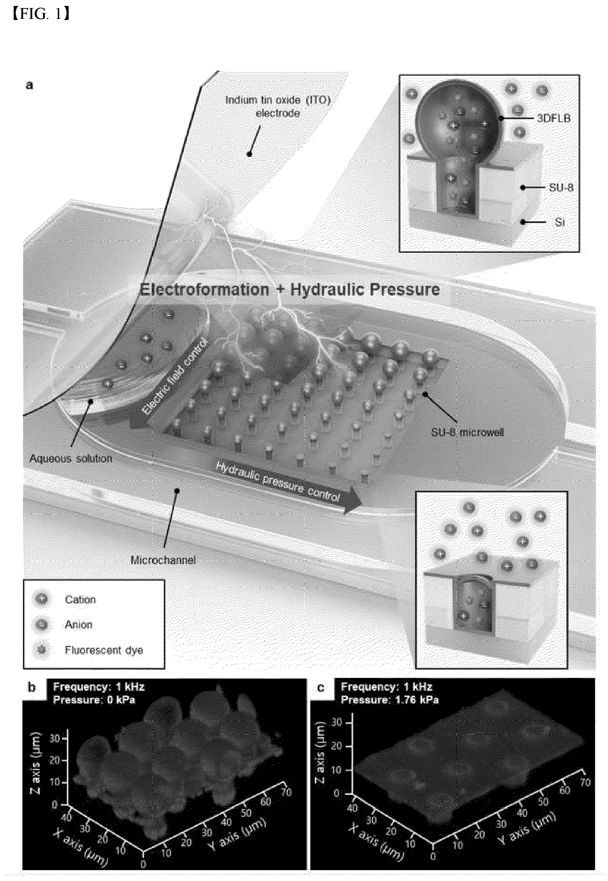

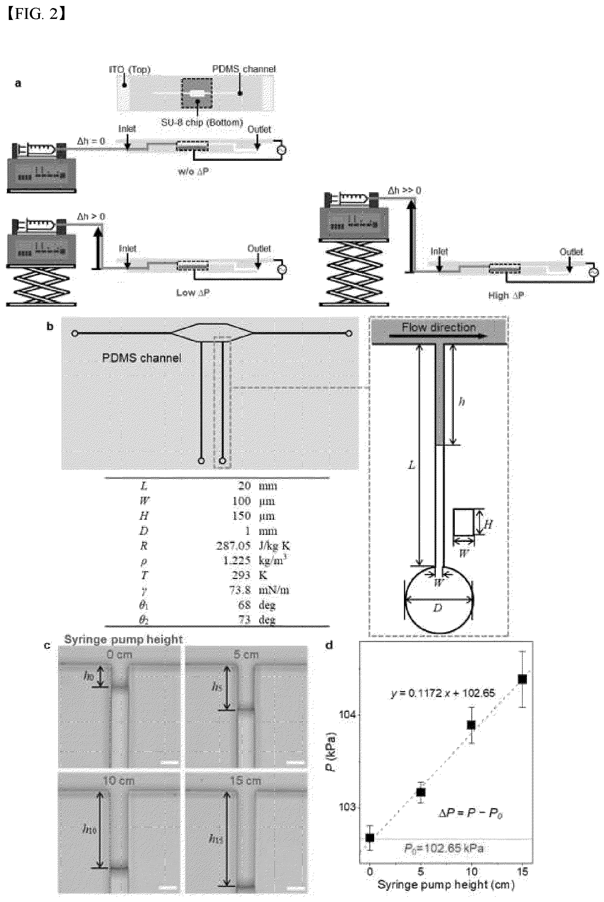

A simple microreactor-embedded straight-line microchannel capable of simultaneously generating, observing and utilizing a 3DFLB was manufactured using a PDMS soft lithography technique (see FIGS. 1 and 2). For the experiment, a lipid-coated SU-8 microwell template was placed in the open space of the lower part, and the upper and lower surfaces of the facing ITO and SU-8 microwell templates were bonded to each other. After a hydration solution was injected into the microchannel using a syringe pump, an alternating current (AC) electric field was applied. As the hydration solution, four types including a sucrose solution (10 mM sucrose), a KCl solution (140 mM KCl 10 mM HEPES), a NaCl solution (140 mM NaCl, 10 mM HEPES), and a DPBS solution (150 mM DPBS) were used. Hydraulic pressure was applied by changing the height of the syringe pump (see FIG. 2). After the 3DFLB was completely grown in all microwells through hydraulic pressure-assisted electroformation, the AC electric field was turned off and a hydration solution including a fluorescent dye (3 $\mu$M Alexa Fluor 488) was injected along the microchannels to confirm the sealing of the 3DFLB.

Evaluation of hydraulic pressure of microchannel by height of syringe pump

A pressure measurement method using air compression was applied to directly measure the hydraulic pressure of the microchannel through the height of an external syringe pump. It is difficult to measure static pressure due to the limited space of the microchannel. Therefore, a simple and cost-effective method capable of integrating closed-ended pressure taps with a small width and height along the length of a microchannel and thus using the pressure taps as a pressure sensor was used. Further details can be found in "Lee, S.-Y. & (Wereley, S. T. Pressure Measurement in Microchannel Using Air Compression. in Fluids Engineering vol. 259 547-552. (ASMEDC, 2003)." The mathematical model is based on the ideal gas law and the difference in interfacial pressure due to the surface tension in microchannels. The pressure of trapped air may be expressed as follows:

$$P_{air} = \frac{m_{air}RT}{V_{compressed}}$$

here, $P_{air}$, $\rho$, $R$, $T$, $m_{air}$ and $V_{compressed}$ are the pressure of air, density of air, gas constant, temperature, mass of air in the pressure tap and volume of air in the pressure tap, respectively. In the steady state, the local pressure (P) balances the difference between trapped air pressure and interfacial pressure. The pressure difference ($P_{diff}$) at the water-air interface is given as follows:

$$P_{diff} = \gamma \left( \frac{1}{R_1} + \frac{1}{R_2} \right) = \gamma \left( \frac{2\cos\theta_1}{W} + \frac{2\cos\theta_2}{H} \right)$$

here, $y$ is the surface tension. $R_1$ and $R_2$ are the main radii of curvature of the interface. $W$ and $H$ are the width and height of the pressure tap, respectively. The contact angle $\theta$ is measured in the solid contact liquid. Therefore, $P_{water}$ may be derived as follows:

$$P = P_{air} - P_{diff} = \frac{m_{air}RT}{V_{compressed}} - \gamma \left( \frac{1}{R_1} + \frac{1}{R_2} \right)$$

A microchannel with two pressure taps was designed and manufactured as shown in FIG. 2B. The hydraulic pressure was then calculated from the above equation using h (displacement of the meniscus in the pressure tap) measured at various heights of the syringe pump (FIGS. 2C and 2D).

The pressure $P_0$ basically applied to the microfluidic system used in this example is 102.65 kPa, the pressure described in the following examples represent only the pressure ($\triangle P$) additionally applied to $P_0$, and the total pressure is $P_0 + \triangle P$.

In-chip controlled pressure-assisted electroformation through hydrogel block

Appropriate amounts of PEGDMA 1000 and PEGDMA 3400 were dissolved in the solution in a 10 mM sucrose solution to obtain mixtures of PEGDMA1000 and PEGDMA3400 at 15:0, 10:5 and 5:10 wt%. The hydrogel solution sandwiched between two pieces of glass with spacers was cured by being irradiated with ultraviolet rays from a UV lamp. The cured hydrogel was cut into sizes of $15 \times 15$ mm$^2$. The cut hydrogel blocks were overlapped with the microwell template before being assembled into microchannels, and then the sequence of forming 3DFLBs without external hydraulic pressure in the same manner was followed.

<Results>

Hydraulic pressure-assisted electroformation of 3DFLB

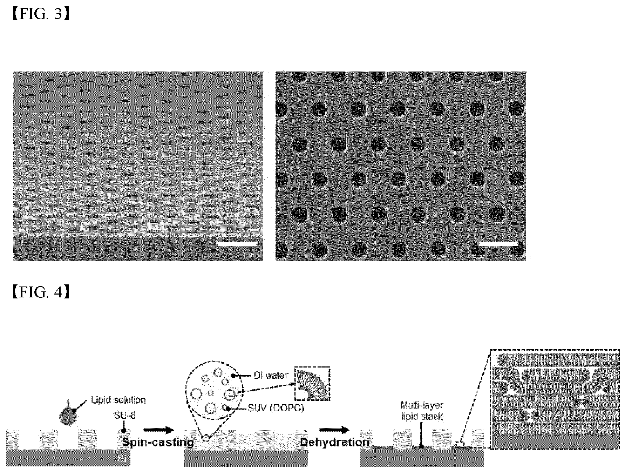

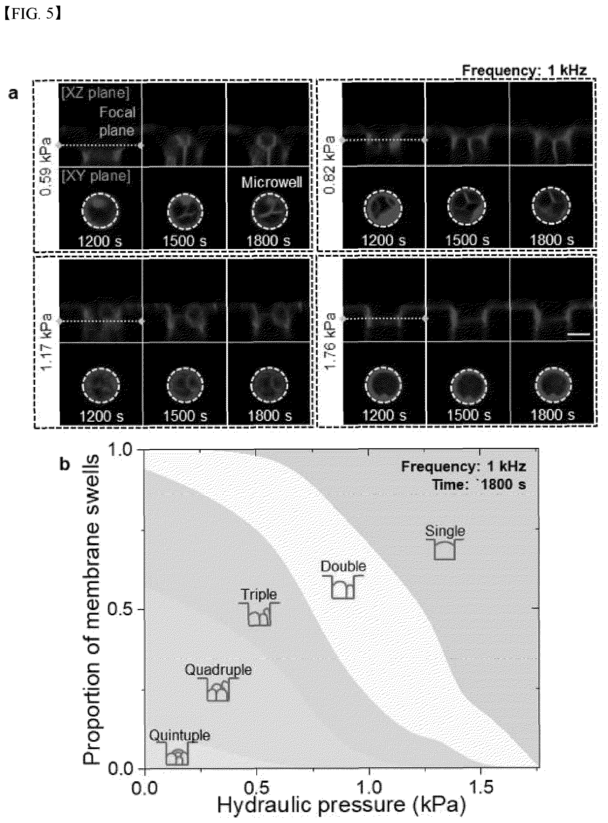

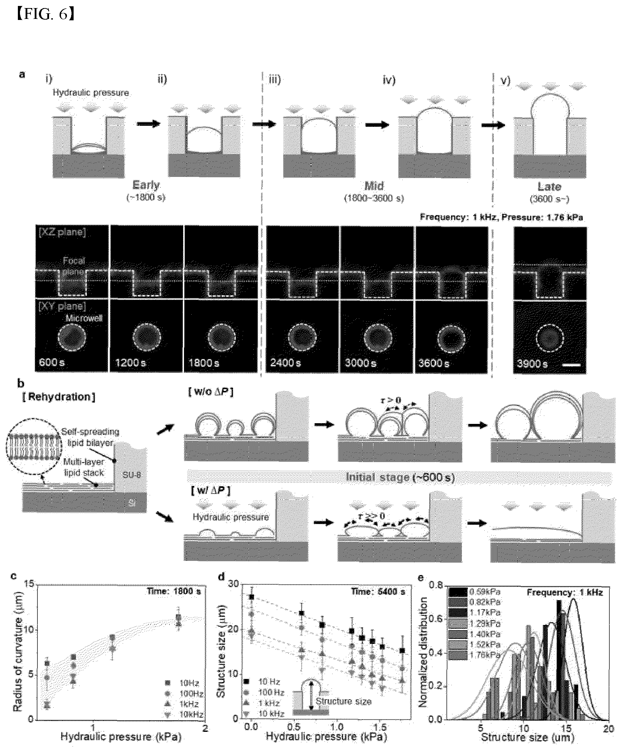

The primary mechanism to generate a 3DFLB includes patterning a lipid into a microwell array (FIG. 3) using spin-casting and dehydration steps and dehydrating a dried lipid stack (FIG. 4) in an aqueous solution in microfluidic channels by applying an electric field. At the same time, the interaction effects of the electric field and hydraulic pressure during the entire period of manufacturing 3DFLBs in non-ionic media were investigated by introducing a finely tuned additional pressure into the microfluidic channel (FIG. 2). FIG. 1A is a schematic view of a 3DFLB whose size and shape can be adjusted according to electric field frequency and pressure. The shape may be gradually adjusted from spherical to cylindrical as it shrinks inside the microwell according to pressure of 0 (FIG. 1B) and 1.76 kPa (FIG. 1C) at the same frequency of 1 kHz (FIG. 5). FIG. 6A shows step-by-step confocal image observation results for the growth of a 3DFLB at a pressure of 1.76 kPa over time. The entire 3DFLB growth period may be classified into three stages: early stage (about 1800 seconds: rapid membrane expansion and fusion), intermediate stage (1800 to 3600 seconds; shape growth), and late stage (3600 seconds or more; growth and sealing saturation). In the case of electroformation (when only an electric field is applied), the membrane expanded explosively, and various membranes expanded to form multiple layers in the initial stage, and then a single bilayer lipid structure was formed in the intermediate stage, but a lipid stack dried under hydraulic pressure (1.76 kPa) expanded and fused in the microwells within the first 600 seconds of the initial stage to immediately form a single multi-layered lipid structure, and then grew to form a 3DFLB in the intermediate stage and reach a saturated state in the late stage. These results indicate that the interaction effect of electric field and pressure mainly occurs in the initial stage. FIG. 2B is a schematic view describing the effect of pressure on microwells in the initial stage. Without hydraulic pressure, individual membrane expansion may sway freely even though the electric field promotes membrane tension ($\tau$), and as a result, membrane fusion or merging occurs due to improved proximity in the confined space of microwells. The promotion of fusion and growth by close adhesion between sufficiently swollen membranes in confined microwells continues from early to intermediate stages until the formation of a single bilayer structure (3DFLB).

On the other hand, when hydraulic pressure is applied, the pressure directly acts on membrane expansion, so that the membrane expansion is forcibly deformed to promote membrane tension, and the single multilayer structures are brought into close proximity to each other within 600 seconds in the sealed microwells. In general, since attached lipid bilayers are affected by long-range static electricity and van der Waals interaction forces, the repulsive force due to dehydration of lipid polar heads dominates other forces at a bilayer distance of about 1.0 to 2.5 nm. For the fusion of lipid membranes, this enormous repulsive force, which depends exponentially on the hydration force at the distance between the membranes, needs to be overcome or reduced to obtain close contact between the bilayers.

In the present invention, membrane fusion occurring rapidly in the early stage of electroformation despite a slight additional pressure of 1.76 kPa or less by reflecting the interaction effect of the confined space of the microwell template, pressure, and electric field, was successfully controlled.

The improvement of fusion by pressure can be confirmed through the fact that the radius of curvature of membrane expansion formed in microwells changes 2-to 4-fold according to the AC frequency at the end of the early stage (about 1800 seconds) (FIG. 6C). It was shown that the frequency dependence of the radius of curvature decreases with increasing pressure, which is proof that membrane expansion is deformed by hydraulic pressure and expanded to enhance fusion.

In addition, according to the present invention, the size of a 3DFLB may be controlled through various AC frequencies and hydraulic pressures (FIG. 6D). According to the Maxwell-Wagner model, lipid membranes act as insulators. Therefore, the charging time of lipid membranes may be shortened by high AC frequencies, leading to AC field effects and a reduction in 3DFLB size. Hydraulic pressure may be smoothly regulated when the 3DFLB expands horizontally and may suppress the size of the structure. FIG. 6E is the size distribution of the final 3DFLBs manufactured at an AC frequency of 1 kHz and various hydraulic pressures.

Membrane fusion control and 3DFLB sealing under physiological ionic conditions

Based on the fact that the fusion function was improved by adding hydraulic pressure during electroformation in non-ionic media, hydraulic pressure effects on 3DFLB array generation and sealing in microfluidic channels under physiological ionic conditions were analyzed.

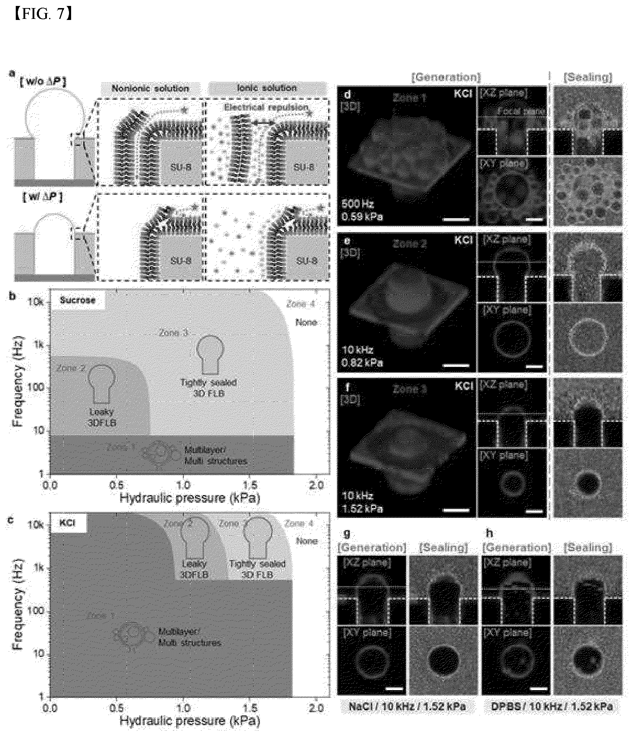

Membrane fusion between the 3DFLB and the self-spreading lipid bilayer on the entire microwell surface is important for tight sealing (complete compartmentalization) of the 3DFLB (FIG. 7A). In general, lipid membranes on solid surfaces rarely fuse with adjacent lipid vesicles due to electrostatic repulsion, which is even more severe in ionic solutions. FIG. 7B shows the relationship between the shape of the lipid structure produced by changes in pressure and AC frequency and the sealing dependence in non-ionic media. An appropriate combination of frequency and hydraulic pressure is required to manufacture a tightly sealed 3DFLB. When hydraulic pressure is applied, the membrane fusion between the 3DFLB and the self-spreading lipid bilayer of the microwell is enhanced, so that the tight sealing region of a firm 3DFLB is extended.

Considering the electrical properties (increase in electrostatic repulsive force according to ion concentration) under physiological conditions containing ions, it is more difficult to generate and control the structure of 3DFLB by electroformation as shown in FIG. 7C. There were considerable changes in each region showing the shape and sealing properties changes of a 3DFLB in a KCl solution according to frequency and hydraulic pressure compared to non-ionic media conditions.

Since the dried lipid stack randomly and spontaneously expands in a physiological KCl buffer (approximately 300 mOsml/L) at low hydraulic pressure and a 500 Hz frequency of Region 1, the individual membrane expansion that initially occurs in the microwell do not fuse and grow on its own, resulting in the formation of leaky multivesicles and multi-layered structures (FIG. 7D). In the case of 10 kHz and 0.82 kPa hydraulic pressure of Region 2, the guidance of appropriately improved membrane expansion and confined space at high frequency produced a 3DFLB. However, leaks may still occur because fusion for sealing requires higher pressure (FIG. 7E). In a physiological KCl solution, a robust 3DFLB could be confirmed only at higher pressure (FIG. 7F).

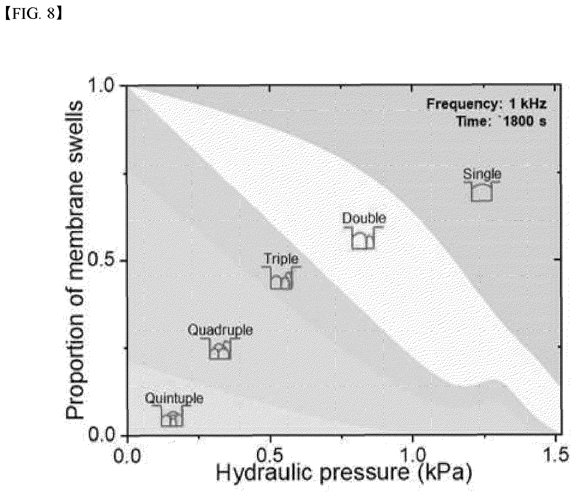

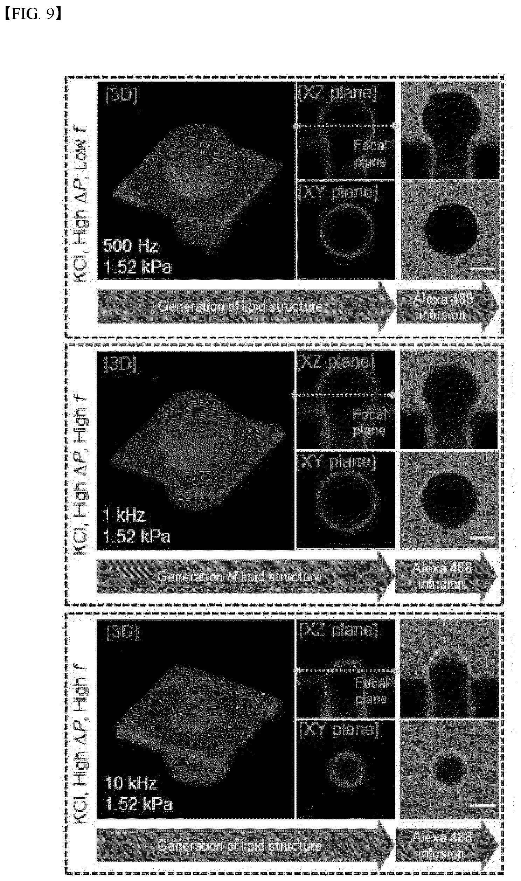

When a sufficiently high hydraulic pressure is combined with an appropriate AC frequency of 500 Hz or higher, the membrane expansion may be fused in the microwells in the early stage (FIG. 8), and the 3DFLB and the self-spreading lipid bilayer may be reliably fused in the KCl solution in the final stage (FIG. 9). The controlled fusion of membrane expansion by hydraulic pressure may also be applied to other physiological buffers such as NaCl and DPBS (FIGS. 7G and 7H). Furthermore, as a result of size distribution analysis of 3DFLBs produced in various buffers (KCl solution, NaCl solution, DPBS, and the like), it was confirmed that a uniform size distribution was shown.

Biological response of 3DFLB immobilized in physiological buffer

The integration of membrane proteins into artificial membranes is essential for model cell membrane analysis and cell-mimetic biosensors and bioreactors. Therefore, the integration of proteins with 3DFLBs generated under controlled pressure and AC frequency was analyzed in a KCl solution. In this experiment, melittin, which is a pore-forming peptide involved in pathological and physiological responses, was integrated into 3DFLB.

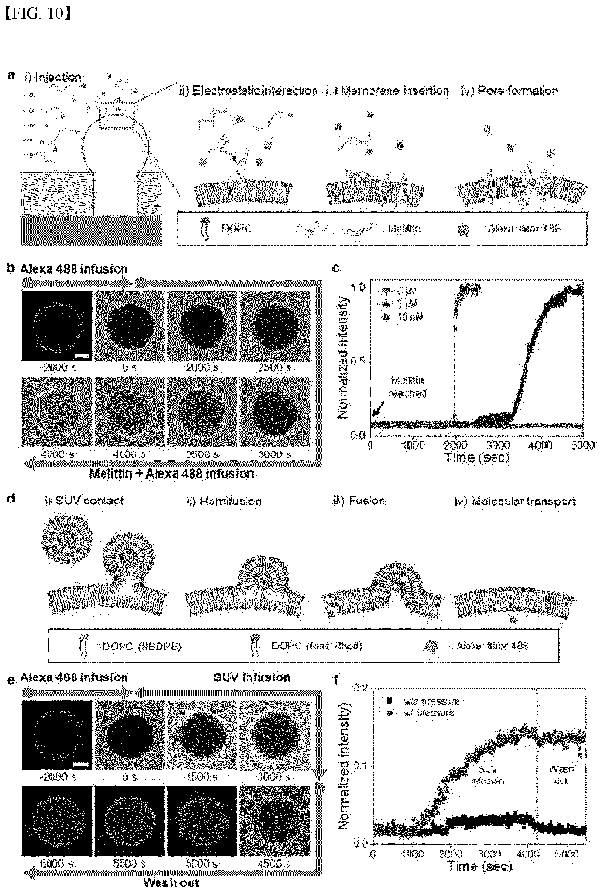

Melittin, a main peptide component of bee venom, is a powerful cytolytic anti-cancer peptide. Melittin binds to lipid membranes within a few milliseconds to take the form of an amphipathic $\alpha$-helix oriented parallel (inactive) or perpendicular (active; pore-forming) to the membrane (FIG. 10A). After a tightly sealed 3DFLB was confirmed in advance, Alexa Fluor 488 (green fluorescence) and melittin were injected into a microfluidic channel to evaluate melittin pore formation and confirm a bilayer membrane (FIG. 10B).

FIG. 10C shows the change in fluorescence intensity of a 3DFLB according to a melittin concentration of 0, 3, and 10 $\mu$M. Since the number of bound melittin monomers and the rate of pore formation increase as the concentration of melittin increases, the onset of transport of dye molecules is accelerated, so that the unilamellar and bio-functionality of a 3DFLB generated at controlled pressure and AC frequency can be confirmed.

Membrane fusion is a ubiquitous biological process, such as egg fertilization, viral infection, and hormone and neurotransmitter release. Vesicular transport or intracellular movement through membrane fusion is an important cellular activity responsible for the movement of molecules between various specific compartments surrounded by membranes. According to the present invention, such vesicular transport may be mimicked by delivering an SUV (about 100 nm) including a fluorescent dye to an immobilized 3DFLB in a microfluidic channel. Membrane fusion for vesicular transport is controlled by components such as SNARE proteins or fusion peptides of biological membranes. Pressure was introduced to control membrane fusion (FIG. 10D). When pressure is applied, the injected SUVs sequentially fuse with the 3DFLB to enhance the fluorescence intensity of the sealed 3DFLB (FIG. 10E). FIG. 10F shows that the fluorescence intensity of the sealed 3DFLB gradually increases compared to the case of no pressure and then remains constant even after washing the external SUV A general SUV-GUV conjugation works better than a GUV-GUV conjugation, but SUV-GUV hardly fuses, meaning that the pressure applied in the present invention can promote the fusion of an SUV and 3DFLB to mimic vesicular transport. Considering that it is important to integrate various cellular components or membrane proteins to prepare synthetic cells or membranes, pressure may become a candidate variable for controlling membrane fusion to mimic cellular activity.

In-chip pressure control and improvement of lifetime of 3DFLB

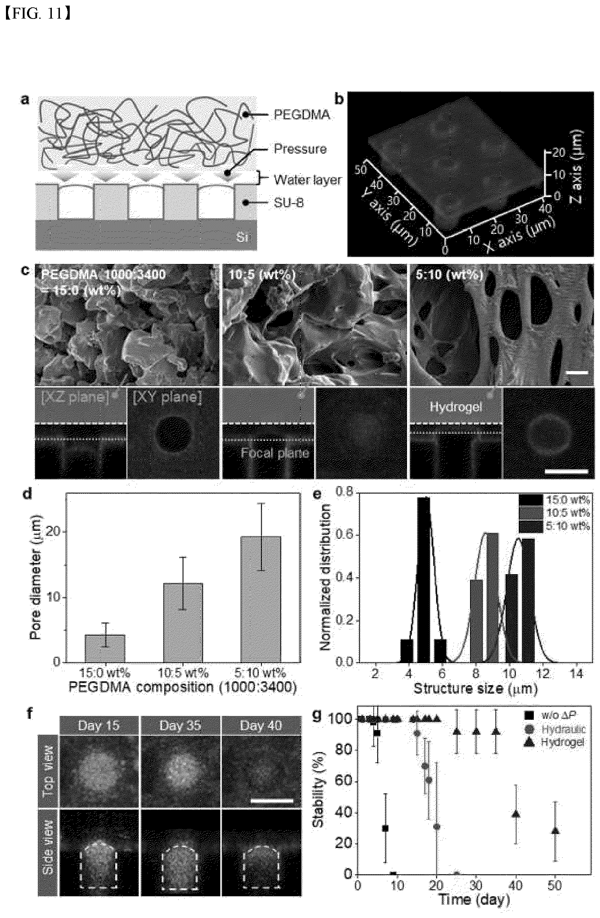

By smoothly controlling the electric field and hydraulic pressure at the same time, it is possible to successfully prepare a 3DFLB with a firm structure. However, when hydraulic pressure is controlled by external equipment, disturbances during the preparation of 3DFLB occur and stability and yield may deteriorate. Therefore, the generation of internal pressure was tested by a method of generating additional pressure in microwells by overlapping hydrogel blocks on a microwell array substrate of a microfluidic channel instead of external pressure control (FIG. 11A). FIG. 11B is a confocal microscope image of a 3DFLB produced by a hydrogel block, proving that a pressure comparable to a hydraulic pressure of 1.76 kPa can be generated to replace hydraulic pressure controlled by external equipment in microfluidic channels. Since the pressure generation by the hydrogel block is affected by the hydrogel pores, the pore size was adjusted by regulating the mixing ratio of two types of hydrogels to 15:0, 10:5, and 5:10 wt%, respectively (FIG. 11C). When a PEGDMA 1000-only hydrogel block is used, a 3DFLB may grow under the microwell opening due to high pressure. In contrast, large pore hydrogel mixtures apply relatively low pressure, so that due to the overlapping hydrogel, a 3DFLB grows and thus spreads over the microwell openings. As the mixing ratio of PEGDMA 3400 increases, the pore diameter of the hydrogel increases from 4.27 to 19.24 $\mu$m to decrease the pressure of the microwell (FIG. 11D), and thus the structural size of the 3DFLB increases (FIG. 11E). Hydrogel blocks may improve the homogeneity of 3DFLBs and also greatly improve the stability of 3DFLBs (FIG. 11F).

It was confirmed that in the case of a 3DFLB in the related art, the lifetime was only 5.5 days, but in the case of the 3DFLB prepared by hydraulic pressure, a stable lifetime lasting at least 17 days was exhibited. Furthermore, stability increased to 38 days when a hydrogel block, which prevents external flow disturbances, was used (FIG. 11G). This improved lifetime of the lipid bilayer structure may be used not only in the laboratory but also in various applications such as commercial artificial cells, cell-mimetic biosensors, and bioreactors.

In summary, a simple and systematic approach for controlling membrane fusion was developed for robust sealing and manufacture of a solvent-free immobilized GUV (3DFLB).

A combination of microwell template-guided self-assembly, electric field and hydraulic pressure was able to form a lipid bilayer structure with a controlled size and shape, monodispersity, and excellent stability and biofunctionality.

The method proposed in the present invention can overcome the limitations of electroformation through membrane fusion control and improve the potential of artificial cell membranes in various application fields. The proposed strategy can be further developed by additionally integrating electrodes into the template, using block copolymers, or integrating other membrane proteins and biomolecules.

According to the present invention, it is possible to easily prepare a 3D biological bilayer membrane structure which is tightly sealed even under physiological ionic conditions by applying pressure during electroformation to improve membrane fusion without biological materials such as proteins or peptides that regulate membrane fusion.

The existing solvent-based artificial cell membrane preparation technology has a problem in that residual solvent remaining in the prepared artificial cell membrane deteriorates the function of a membrane protein that binds to the artificial cell membrane.

In addition, electroformation, which is almost the only method capable of preparing a 3D artificial cell membrane among solvent-free preparation methods, cannot prepare a biological membrane structure with a uniform size in a physiological solution that is a condition in which ions similar to those of living organisms are included due to the characteristics of the preparation method that applies an electric field, and furthermore, when a biological membrane structure in the form of being immobilized on a substrate is prepared under the condition of a physiological solution, the structure cannot be tightly sealed, so that there is a problem in that various applications such as drug screening and biosensors, which are performed through biosignals, material delivery, and the like, cannot be performed by distinguishing between the inside and outside of the biological membrane structure.

The present invention provides a method for preparing a tightly-sealed three-dimensional biological bilayer membrane structure in the form of being immobilized on a substrate, and according to the present invention, 1) solvent-free, 2) physiological solution, 3) form immobilized on a substrate, 4) tight sealing, and 5) excellent durability can all be satisfied. The present invention relates to the preparation of a 3D biological bilayer membrane structure that is highly sealed and very stable (maintains stability for 17 to 38 days or more).

More specifically, according to the present invention, it is possible to provide a 3D biological bilayer membrane structure, which can more effectively sense biological signals by preventing leakage that occurs when a 3D structure consisting of a lipid membrane in the related art is formed on a substrate to maintain a tight sealing, and has high stability and long lifetime by enabling perfect sealing in a physiological solution.

Furthermore, according to the present invention, a lifetime of about 38 days or more can be secured by remarkably increasing the lifetime of the 3D biological bilayer membrane structure. Accordingly, there is an advantage in that the stability, which is one of the obstacles to application and industrialization, can be significantly increased.

Further, the present invention has an effect capable of mimicking a biological signal sensing function, which is the core function of the cell membrane, through a biological bilayer membrane with a biological mimetic three-dimensional structure having the structural and/or functional characteristics of the cell membrane that constitutes the cell.

**Claims**

1. A method for preparing a three-dimensional (3D) biological bilayer membrane structure, the method comprising:
preparing a microwell array in which multiple microwells are formed on a substrate (Step a);

    coating the inside of the microwells with an artificial biological membrane material (Step b); and
    injecting a buffer solution onto the microwell coated with the biological membrane material and then applying an electric field and pressure (Step c).

2. The method of claim 1, wherein the microwell has a diameter in a range of 1 to 20 $\mu$m.

3. The method of claim 1, wherein the microwell has an aspect ratio (aspect ratio = depth/diameter) in a range of 0.2 to 10.0.

4. The method of claim 1, wherein the artificial biological membrane material comprises one or more of a lipid or a block copolymer.

5. The method of claim 1, wherein the buffer solution is a physiological solution.

6. The method of claim 5, wherein the physiological solution comprises one or more selected from the group consisting of a solution containing $H^+$, $K^+$, $Na^+$ or $Cl^-$ ions, 4-(2-hydroxyethyl)-1-piperazineethanesulfonic acid (HEPES), tris(hydroxymethyl)aminomethane (Tris) or Dulbecco's phosphate-buffered saline (DPBS).

7. The method of claim 1, wherein the electric field is applied by applying an electric field using an electroformation method.

8. The method of claim 1, wherein the pressure is applied by external equipment, or by a method of generating relative pressure in the microwells by interfering with the flow of fluid on a microwell array substrate.

9. The method of claim 8, wherein the method of generating pressure in the microwell by interfering with the flow of fluid is a method of generating pressure by injecting a hydrogel block.

10. The method of claim 9, wherein the hydrogel comprises polyethylene glycol dimethacrylate (PEGDMA).

11. The method of claim 9, wherein the hydrogel is a mixture of PEGDMA 1000 and PEGDMA 3400.

12. The method of claim 1, wherein Step c is performed under conditions of a frequency of 5 Hz to 10 MHz and a pressure of 300 kPa or less.

13. A 3D biological bilayer membrane structure prepared according to claim 1.

14. The 3D biological bilayer membrane structure of claim 13, wherein the structure maintains 90% or more stability for at least 17 days.

【FIG. 1】

【FIG. 2】

【FIG. 3】

【FIG. 4】

【FIG. 5】

【FIG. 6】

EP 4 344 845 A1

【FIG. 7】

18

【FIG. 8】

【FIG. 9】

【FIG. 10】

【FIG. 11】

**EP 4 344 845 A1**

# EUROPEAN SEARCH REPORT

Application Number

EP 23 20 0679

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/087807 A1 (KIM TAE SONG [KR] ET AL) 19 March 2020 (2020-03-19) | 1-8, 12-14 | INV.<br>B29C33/42 |
| A | * paragraphs [0042], [0047], [0054], [0057], [0059], [0071], [0075]; claims 1,4,5,14-16 * | 9-11 | B29C39/26<br>B29C39/42<br>C25D1/00<br>G01N33/554 |
| | ----- | | G01N33/92 |
| A | WEINBERGER ANDREAS ET AL: "Gel-Assisted Formation of Giant Unilamellar Vesicles", BIOPHYSICAL JOURNAL, ELSEVIER, AMSTERDAM, NL,<br>vol. 105, no. 1, 2 July 2013 (2013-07-02), pages 154-164, XP028576982,<br>ISSN: 0006-3495, DOI: 10.1016/J.BPJ.2013.05.024<br>* the whole document * | 1-14 | |
| | ----- | | |
| A | US 2012/025414 A1 (SCHMIDT JACOB J [US] ET AL) 2 February 2012 (2012-02-02)<br>* paragraph [0036] – paragraph [0042] *<br>* paragraph [0066] – paragraph [0070] *<br>* paragraph [0079] – paragraph [0087] * | 1-14 | |
| | ----- | | |

TECHNICAL FIELDS
SEARCHED      (IPC)

B29C
C23C
G01N
C25D

The present search report has been drawn up for all claims

| Place of search | Date of completion of the search | Examiner |
|---|---|---|
| Berlin | 14 February 2024 | Cubas Alcaraz, Jose |

## ANNEX TO THE EUROPEAN SEARCH REPORT
## ON EUROPEAN PATENT APPLICATION NO.

EP 23 20 0679

This annex lists the patent family members relating to the patent documents cited in the above-mentioned European search report.
The members are as contained in the European Patent Office EDP file on
The European Patent Office is in no way liable for these particulars which are merely given for the purpose of information.

14-02-2024

| Patent document cited in search report | | Publication date | Patent family member(s) | | Publication date |
|---|---|---|---|---|---|
| US 2020087807 | A1 | 19-03-2020 | KR | 102013337 B1 | 21-10-2019 |
| | | | US | 2020087807 A1 | 19-03-2020 |
| US 2012025414 | A1 | 02-02-2012 | NONE | | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20220125837

- KR 20230106268

**Non-patent literature cited in the description**

- **LEE, S.-Y. ; WERELEY, S. T.** Pressure Measurement in Microchannel Using Air Compression. *Fluids Engineering,* vol. 259, 547-552